# EUROPÄISCHE PATENTSCHRIFT

(11) **EP 0 502 372 B1**
(45) Veröffentlichungstag und Bekanntmachung des Hinweises auf die Patenterteilung: **17.05.1995**
(21) Anmeldenummer: 92102891.6
(22) Anmeldetag: 21.02.1992
(51) Int. Cl.: C07C 43/307, C25B 3/02

(54) **4-tert-Alkyl-2-Methylbenzaldehyddialkylacetale**
4-tert-Alkyl-2-Methylbenzaldehydedialkylacetals
Dialkylacétals de 4-tert-alkyl-2-méthylbenzaldéhydes

(30) Priorität: 02.03.1991 DE 4106661
(43) Veröffentlichungstag der Anmeldung: 09.09.1992
(73) Patentinhaber: BASF Aktiengesellschaft, 67063 Ludwigshafen (DE)
(72) Erfinder: Hermeling, Dieter, Dr., W-6710 Frankenthal (DE)

(56) Entgegenhaltungen:
- EP-A- 0 012 240
- EP-A- 0 287 954
- DE-A- 3 713 732

## Beschreibung

Die vorliegende Erfindung betrifft neue 2-Methylbenzaldehyddialkylacetale, ihre Herstellung sowie ihre Verwendung als lagerstabile Depotverbindungen für substituierte 2-Methylbenzaldehyde.

Aus EP-A-12 240 und DE-A-37 13 732 sind Benzaldehyddialkylacetale bekannt, die durch anodische Oxidation substituierter Toluole herstellbar sind. Aus EP-A-12 240 geht ebenfalls hervor, daß sowohl ortho-, als auch meta- und para-substituierte Toluole zu den entsprechenden substituierten Benzaldehyddialkylacetalen oxidiert werden können. Dies bedeutet, daß im Falle von 4-substituierten 1,2-Dimethylbenzolen ein Produktgemisch bestehend aus einem 4-substituierten 2-Methylbenzaldehyddialkylacetal und einem 5-substituierten 2-Methylbenzaldehyddialkylacetal entstehen sollte.

Benzaldehyde sind aufgrund ihrer leichten Oxidierbarkeit nur eingeschränkt lagerstabil. Wesentlich stabilere Lagerverbindungen sind Benzaldehyddialkylacetale, aus denen die Benzaldehyde durch Hydrolyse in nahezu quantitativen Ausbeuten erhalten werden.

Benzaldehyde finden z.B. nach Ullmann's Encyklopädie der technischen Chemie, 4. Auflage, Band 20, s. 234 bis 236 Verwendung als Riechstoffe oder Riechstoffvorprodukte. Ebenso dienen sie als Vorprodukte für Pflanzenschutzmittel, Farbstoffe oder Pharmaka. Die Verwendung speziell von 4-substituierten 2-Methylbenzaldehyden als Pharmavorprodukt wird z.B. in BE-A 816 463 erwähnt.

Nach DE-A 37 13 732 finden auch Benzaldehyddialkylacetale in der Parfümerie Verwendung. Ihr Vorteil gegenüber den freien Aldehyden ist ihre erheblich größere Stabilität in nicht sauren Medien.

Der vorliegenden Erfindung lag die Aufgabe zugrunde, neue Verbindungen zu finden, die zu verbesserten Wirkstoffen bzw. die zu verändernden Riechstoffnuancen führen.

Demgemäß wurden 4-tert.-Butyl- und 4-tert.-Amyl-2-methylbenzaldehyddimethylacetal gefunden sowie deren Herstellung und deren Verwendung zur Herstellung von 2-Methylbenzaldehyden.

Nach dem erfindungsgemäßen Verfahren lassen sich die beiden 4-substituierten 2-Methylbenzaldehyddialkylacetale besonders vorteilhaft aus leicht zuganglichen 4-substituierten 1,2-Dimethylbenzolen einstufig in hohen Ausbeuten und Selektivitäten herstellen.

Ein weiterer Vorteil dieses Verfahrens besteht darin, daß keine aufwendigen Aufreinigungsmaßnahmen erforderlich sind, da die schwer abtrennbaren stellungsisomeren 5-substituierten 2-Methylbenzaldehyddialkylacetale stark zurückgedrängt werden.

Die neuen Verbindungen lassen sich durch elektrochemische Verfahren in den technisch üblichen Elektrolysezellen herstellen. Bevorzugt werden ungeteilte Durchflußzellen verwendet.

Als Anoden kommen z.B. Edelmetallelektroden wie Platin oder Oxidelektroden wie Ti/RuOₓ, RuO₂ oder Cr₂O₃ in Frage. Bevorzugtes Anodenmaterial ist Graphit.

Als Kathoden kommen z.B. Stahl, Eisen, Nickel, Kupfer, Zink und Kohle sowie Edelmetalle wie Platin in Betracht. Bevorzugtes Kathodenmaterial ist Graphit.

Der Elektrolyt setzt sich aus der jeweiligen Ausgangsverbindung, Methanol und einem Hilfselektrolyten zusammen.

Als Hilfselektrolyte kommen Neutralsalze, Säuren und Basen in Betracht. Beispiele für Neutralsalze sind Fluoride wie KF, Sulfonate wie NaSO₃Ph, Sulfate wie (CH₃)₄NSO₄CH₃, Tetrafluoroborate wie NaBF₄, Phosphate und Phosphonate. Beispiele für Säuren sind Schwefelsäure, Alkyl- und Arylsulfonsäuren wie Methyl- oder Benzolsulfonsäure. Als Basen dienen z.B. Alkoholate wie NaOCH₃ oder Hydroxide wie KOH.

Der Elektrolyt hat beispielsweise folgende Zusammensetzung:
1 bis 49, vorzugsweise 5 bis 30 Gew.% Ausgangsverbindung
50 bis 99, vorzugsweise 70 bis 95 Gew.% Methanol
0,1 bis 5, vorzugsweise 0,2 bis 3 Gew.% Hilfselektrolyt.

Die Stromdichte kann bei dem erfindungsgemäßen Verfahren in weiten Grenzen, z.B. von 0,5 bis 25 A/dm², bevorzugt von 1 bis 10 A/dm² gewählt werden.

Auch die Temperaturen können in weiten Grenzen variiert werden. So können die Oxidationen bei 0 bis 100°C, bevorzugt bei 20 bis 80°C durchgeführt werden.

Es wird im allgemeinen bei Temperaturen unterhalb des Siedepunktes des Methanols gearbeitet.

Die Elektrolysen werden bevorzugt unter Normaldruck durchgeführt, es kann aber auch unter Druck von 0,09 bis 10 bar elektrolysiert werden. Durch die damit verbundene Siedepunktserhöhung kann auch in Methanol bei Temperaturen oberhalb von 60°C elektrolysiert werden.

Die Ausgangsverbindungen lassen sich weitestgehend umsetzen. Nicht umgesetztes 4-substituiertes 1,2-Dimethylbenzol und der als Zwischenprodukt auftretende 4-substituierte 2-Methylbenzylmethylether können in die Elektrolyse zurückgeführt werden. Die Elektrolyse kann sowohl kontinuierlich als auch diskontinuierlich durchgeführt werden. Die Aufarbeitung der Elektrolyseausträge erfolgt nach konventionellen Methoden, vorzugsweise wird destillativ aufgearbeitet.

Die neuen 2-Methylbenzaldehyddialkylacetale dienen z.B. als Depotverbindungen für 2-Methylbenzaldehyde. Sie lassen sich leicht zu den entsprechenden Benzaldehyden hydrolysieren. Die Verseifung der Diacetale nimmt man nach an sich bekannten Methoden vor, beispielsweise durch einfaches Erhitzen in Wasser unter Zusatz katalytischer Mengen Säure auf Temperaturen von 40 bis 95°C.

Die erfindungsgemäßen Verbindungen haben folgende Geruchsnoten:
- 4-tert.-Butyl-2-methylbenzaldehyddimethylacetal:
   tabak-lederartig phenolischer Geruch
- 4-tert.-Amyl-2-methylbenzaldehyddimethylacetal:
   süß-phenolisch, minzig-fruchtiger Geruch mit Ingwernote
Die aus der DE-A-37 13 732 bekannte Geruchsnoten für Benzaldehyddialkylacetale sind:
- Benzaldehyddimethylacetal: süßlich grün
- Benzaldehyddiethylacetal: süß, wild und grün
- Anisaldehyddimethylacetal: herb-grün, etwas blumig
- Anisaldehyddiethylacetal: blumig-süß, leicht grün
- 3-tert.-Butyl-4-methoxybenzaldehyddimethylacetal:
   blumig-süß mit erdiger Nebennote
- 3-tert.-Butyl-4-methoxybenzaldehyddiethylacetal:
   langhaftende Grünnote.

### Beispiele

### Beispiel A (nicht erfindungsgemäß)

Elektrosynthese von 4-Methoxy-2-methylbenzaldehyddimethylacetal ungeteilte Zelle mit 11 bipolaren Elektroden Graphit

| | |
|---|---|
| Apparatur: | 250 g (1,838 Mol) 4-Methoxy-1,2-dimethylbenzol, |
| Anoden: | |
| Elektrolyt: | 30 g NaSO₃C₆H₅, 2750 g Methanol |
| Kathoden: | Graphit |
| Stromdichte: | 3,4 A/dm² |
| Elektrolysetemperatur: | 23°C |

### Elektrolyse mit 4,75 F/mol 4-Methoxy-1,2-dimethylbenzol

Der Elektrolyt wird während der Elektrolyse mit 200 l/h durch die Zelle gepumpt. Nach Beendigung der Elektrolyse wird das Methanol bei Normaldruck bis zu einer Sumpftemperatur von 120°C abdestilliert, das Leitsalz abfiltriert und das Filtrat im Vakuum reindestilliert. Man erhält 193 g (54 %) 4-Methoxy-2-methylbenzaldehyddimethylacetal; Sdp.: 130°C/6 mbar.

### Beispiel 1 (Beispiel 1-3 sind erfindungsgemäß

### Elektrosynthese von 4-tert.-Butyl-2-methylbenzaldehyddimethylacetal

4-tert.-Butyl-1,2-dimethylbenzol wird in der in Beispiel A beschriebenen Elektrolysezelle unter den dort angegebenen Bedingungen mit 9 bipolaren Elektroden oxidiert.

| | |
|---|---|
| Elektrolyt: | 4317 g (26,648 Mol) 4-tert.-Butyl-1,2-dimethylbenzol, 144 g KSO₃C₆H₅ und 24 320g Methanol |
| Elektrolysetemperatur: | 36°C |

### Elektrolyse mit 5,8 F/mol 4-tert.-Butyl-2-methylbenzaldehyddimethylacetal

Die Aufarbeitung des Elektrolyten erfolgt wie in Beispiel A beschrieben. Nach Reindestillation im Vakuum werden 4065 g (69 %) 4-tert.-Butyl-2-methylbenzaldehyddimethylacetal erhalten;
Sdp.: 100 bis 105°C/4 mbar.

### Beispiel 2

### Elektrosynthese von 4-tert.-Amyl-2-methylbenzaldehyddimethylacetal

4-tert.-Amyl-1,2-dimethylbenzol wird in der in Beispiel A beschriebenen Elektrolysezelle unter den dort angegebenen Bedingungen oxidiert.

| | |
|---|---|
| Elektrolyt: | 121 g (0,688 Mol) 4-tert.-Amyl-1,2-dimethylbenzol, 15 g NaSO₃C₆H₅ und 2864 g Methanol |
| Elektrolysetemperatur: | 25°C |

### Elektrolyse mit 6 F/mol 4-tert.-Amyl-1,2-dimethylbenzol

Die Aufarbeitung erfolgt wie in Beispiel A beschrieben. Nach Reindestillation im Vakuum werden 12,1 g (10 %) nicht umgesetztes Edukt, 21,9 g (15 %) 4-tert.-Amyl-2-methylbenzylmethylether und 63,3 g (39 %) 4-tert.-Amyl-2-methylbenzaldehyddimethylacetal erhalten; Sdp.: 124-126°C/8 mbar.

### Beispiel 3

### Herstellung von 4-tert.-Amyl-2-methylbenzaldehyd

56 g 4-tert.-Amyl-2-methylbenzaldehyddimethylacetal werden 1 h lang mit 170 g 1 %iger Schwefelsäure gekocht. Das gebildete Methanol wird abdestilliert, die organische Phase abgetrennt und über Na₂SO₄ getrocknet. Man erhält 35 g (80 %) 4-tert.-Amyl-2-methylbenzaldehyd.

## Patentansprüche

1. 4-tert.-Butyl- oder 4-tert.-Amyl-2-methylbenzaldehyddimethylacetal.

2. Verfahren zur Herstellung von 4-tert.-Butyl- oder 4-tert.-Amyl-2-methylbenzaldehyddimethylacetal gemäß Anspruch 1, dadurch gekennzeichnet, daß man 4-tert.-Butyl- bzw. 4-tert.-Amyl-1,2-dimethylbenzol mit Methanol in Gegenwart eines Hilfselektrolyten elektrochemisch oxidiert.

3. Verwendung von 4-tert. -Butyl- oder 4-tert.-Amyl-2-methylbenzaldehyddialkylacetal gemäß Anspruch 1 als lagerstabile Depotverbindungen zur Herstellung von 4-tert.-Butyl- bzw. 4-tert.-Amyl-2-methylbenzaldehyd.

## Claims

1. 4-tert-Butyl- or 4-tert-amyl-2-methylbenzaldehyde dimethyl acetal.

2. A process for the preparation of 4-tert-butyl- or 4-tert-amyl-2-methylbenzaldehyde dimethyl acetal as claimed in claim 1, wherein 4-tert-butyl- or 4-tert-amyl-1,2-dimethylbenzene, respectively, is electrochemically oxidized with methanol in the presence of an auxiliary electrolyte.

3. Use of a 4-tert-butyl- or 4-tert-amyl-2-methylbenzaldehyde dialkyl acetal as claimed in claim 1 as a depot compound, having a long shelf life, for the preparation of 4-tert-butyl- or 4-tert-amyl-2-methylbenzaldehyde, respectively.

## Revendications

1. 4-Tert-butyl- ou 4-tert-amyl-2-méthylbenzaldéhydediméthylacétal.

2. Procédé de préparation du 4-tert-butyl- ou 4-tert-amyl-2-méthylbenzaldéhydediméthylacétal suivant la revendication 1, caractérisé en ce que l'on oxyde électrochimiquement le 4-tert-butyl- ou 4-tert-amyl-1,2-diméthylbenzène avec le méthanol en présence d'un électrolyte adjuvant.

3. Utilisation du 4-tert-butyl- ou 4-tert-amyl-2-méthylbenzaldéhydediméthylacétal suivant la revendication 1, à titre de composé dépôt stable à la conservation pour la préparation du 4-tert-butyl- ou 4-tert-amyl-2-méthylbenzaldéhyde.
